# EUROPÄISCHE PATENTSCHRIFT

(11) **EP 2 859 855 B1**
(45) Veröffentlichungstag und Bekanntmachung des Hinweises auf die Patenterteilung: **05.04.2017**
(21) Anmeldenummer: 13187700.3
(22) Anmeldetag: 08.10.2013
(51) Int. Cl.: A61B 17/29, A61B 17/28, A61B 17/80, A61B 17/88, A61M 25/02

(54) **Repositionszange mit zweifacher 90°-Verformung zur Verteilung auf zwei Ebenen**
Repositioning pliers with dual 90° deformation for distribution on two levels
Pince de repositionnement doté d'une déformation double à 90° pour la répartition sur deux plans

(43) Veröffentlichungstag der Anmeldung: 15.04.2015
(73) Patentinhaber: Karl Leibinger Medizintechnik GmbH & Co. KG, 78570 Mühlheim/Donau (DE)
(72) Erfinder: Kohler, Klaus, 72505 Hausen (DE)
(74) Vertreter: Winter, Brandl, Fürniss, Hübner, Röss, Kaiser, Polte - Partnerschaft mbB

(56) Entgegenhaltungen:
- WO-A1-2006/049960
- US-A- 5 059 198
- US-A1- 2004 006 371
- US-A1- 2013 144 313

## Beschreibung

Die Erfindung betrifft eine Repositionszange mit einer ersten Stange und einer zweiten Stange, die an einem eine Drehachse definierenden Drehgelenk zueinander verschwenkbar oder verdrehbar gelagert sind, wobei die erste Stange auf einer Seite des Drehgelenks ein erstes Maulteil, wie eine erste Branche, aufweist und die zweite Stange auf der gleichen Seite des Drehgelenks eine zum Zusammenwirken mit dem ersten Maulteil vorgesehenes zweites Maulteil, wie eine zweite Branche, aufweist, wobei an den beiden Maulteilen Kontaktpunkte zum Kontaktieren eines Knochens oder einer Knochenplatte definiert sind.

Aus dem Stand der Technik, etwa der US 2004/0006371 A1, der WO 2006/049960 A1, der US 2013/0144313 A1, der US 5,059,198 A, der DE 87 02 813 U1 oder der DE 86 12 466 U1 sind bereits ähnliche chirurgische Zangen bekannt. Die DE 101 96 657 B4 offenbart eine Zange zum Eingriff an einem Unterkieferwinkel auf beiden Seiten eines Bruchs, bestehend aus einem ersten Element und einem zweiten Element, welche an einem Gelenk zusammengefügt sind und Griffe auf einer Seite jedes Elements aufweisen, und welche auf der anderen Seite dieses Elements geradlinige Abschnitte in der Nähe des Gelenks und gebogene Abschnitte aufweisen, welche von Vorderenden der geradlinigen Abschnitte hervorstehen und Spitzen aufweisen, die als Besonderheit aufweisen, dass der gebogene Abschnitt des ersten Elements die Spitze aufweist, welche in einer Richtung parallel zur Achse des Gelenks weist und der Biegeabschnitt des zweiten Elements eine Spitze und einen Verlängerungsabschnitt aufweist.

Die EP 1 811 909 B1 offenbart eine intravaginale Vorrichtung zum Behandeln der Gebärmuttererkrankung eines weiblichen Patienten durch Schließen wenigstens einer der Gebärmutterarterien des Patienten. Sie weist ein erstes Klemmelement und ein zweites Klemmelement auf. Das erste Klemmelement weist einen länglichen Griff mit einem proximalen Griffbereich auf, der dazu ausgelegt ist, sich während der Behandlung aus der Patientin heraus zu erstrecken, und der von einem Bediener gehandhabt wird, welcher einen distalen Griffbereich mit einem Schwenkpunkt hat, und welcher dazu ausgelegt ist, sich in einer Ebene um den Schwenkpunkt zu drehen, und eine offene, paddelförmige Backe aufweist, die an dem distalen Griffbereich gesichert ist, die eine distale Spitze mit einer Druck ausübenden Fläche hat, die eine gewebeaufnehmende Ausnehmung proximal zu der Druck ausübenden Fläche hat, wobei ein Paar in Längsrichtung ausgerichteter Seiten, die proximal zu der distalen Spitze liegen, teilweise die Gewebe aufnehmende Ausnehmung definiert, wobei eine erste der in Längsrichtung ausgerichteten Seite mit dem distalen Griffbereich fluchtet und eine zweite der in Längsrichtung ausgerichteten Seite von der ersten in Längsrichtung ausgerichteten Seiten beabstandet ist.

Auch die CN 201806774 U1 offenbart eine Zange zum Einsatz an einem Knochen.

Eine ähnliche Zange ist auch aus der CN 202515727 U bekannt.

Eine Repositionszange ist ferner aus der DE 83 23 877 U1 bekannt, bei der zwei Zangenbranchen fest zentriert oder zu einer sich fest zentrierenden Zange zusammensetzbar und ohne Werkzeuge lösbar sind, wobei die Mäuler beider Zangenbranchen unterschiedlich groß und unterschiedlich ausgeformt sind.

Eine Repositionszange zum Kontaktieren einer Knochenplatte und ein Verfahren zu deren Einsatz ist aus der WO 2010/014719 A1 bekannt.

Die bisherigen Repositionszangen stehen jedoch in vielen Positionen bei der Knochenreposition zu weit vom Knochen ab. Dies kann zu Verletzungen am Weichgewebe führen, da dieses verdrängt werden muss. Wenn mehr Platz für die Repositionszange zur Verfügung gestellt werden soll, muss der Knochen mehr vom Weichgewebe freipräpariert werden bzw. mehr Weichgewebe vom Knochen entfernt werden. Die Wunde wird somit größer. Auch ist die Verwendbarkeit bisheriger Zangen in unterschiedlichen Positionen eingeschränkt oder gar nicht gegeben.

Mit anderen Worten, sind die bisherigen Repositionszangen zu ausladend geformt, so dass Weichgewebe bei minimaler Präparation verletzt wird bzw., um dies zu verhindern, der Knochen im größeren Umfang freipräpariert werden muss.

Es ist daher die Aufgabe der vorliegenden Erfindung, die Nachteile aus dem Stand der Technik zu vermeiden und eine belastbare, kostengünstige, vielseitige und präzise zu nutzende Repositionszange zur Verfügung zu stellen.

Diese Aufgabe erfindungsgemäß dadurch gelöst, dass die Kontaktpunkte in einer gemeinsamen Schwenkebene, die das Drehgelenk beinhaltet, bewegbar sind, in der die Drehachse senkrecht steht und wenigstens eines der Maulteile oder Branchen in Richtung einer zur Schwenkebene parallelen Ebene, etwa einer Parallelebene, abschnittsweise versetzt ist.

Eine solche Repositionszange legt sich besser an die Kontur des Knochens an. Dieses bessere Anlegen liegt auch beim Auswinkeln/Abwinkeln der Zange vor.

Es wird somit ein mehrfach abgewinkeltes unteres Maulteil der Zange zur Verfügung gestellt. Im Prinzip ist das untere Maulteil ca. 90° geformt, wobei die 90°-Formung durch einen Verbindungssteg auf zwei Ebenen verteilt ist.

Vorteilhafte Ausführungsformen sind in den Unteransprüchen beansprucht und werden nachfolgend näher erläutert.

So ist es von Vorteil, wenn nur eine der beiden Maulteile in Richtung der zur Schwenkebene parallelen Ebene verlaufend, also der Parallelebene, ausgerichtet ist. Das andere der beiden Maulteile, bspw. die zweiten Stange mit dem zweiten Maulteil, ist dann gerade ausgestaltet oder zumindest in einer die Längsachse der zweiten Stange und/oder des zweiten Maulteils aufnehmenden Bearbeitungsebene, die deckungsgleich mit der Schwenkebene sein kann, angeordnet. Die Herstellung einer solchen Hälfte der Repositionszange ist dann besonders kostengünstig.

Natürlich ist es auch möglich, dass beide Maulteile die Schwenkebene verlassen und in sie wieder zurückkehren.

Es ist ferner von Vorteil, wenn ein vorzugsweise gerader Abschnitt in der zur Schwenkebene parallelen Ebene verlaufend angeordnet ist. Der gerade Abschnitt ist dann ein Bestandteil des jeweiligen Maulteils, etwa des ersten Maulteils.

Vorteilhaft kann es sein, wenn die Endbereiche, also die Bereiche der freien Enden der beiden Maulteile zueinander parallel und über-/untereinander in Flucht ausgerichtet sind.

Ein vorteilhaftes Ausführungsbeispiel ist auch dadurch gekennzeichnet, dass das erste Maulteil als eine untere Branche und das weitere Maulteil als eine obere Branche ausgestaltet ist, wobei ein freies Ende der unteren Branche in der Schwenkebene betrachtet unterhalb der oberen Branche angeordnet ist. Das angesprochene in Flucht bringen wird dann vereinfacht.

Wenn das zweite Maulteil vollständig oder zumindest überwiegend in der Schwenkebene angeordnet ist, so wird das Auftreten von Drehmomenten im Wesentlichen verhindert.

Es ist ferner zweckmäßig, wenn das zweite Maulteil in der Schwenkebene zwischen dem Drehgelenk und dem freien Ende des zweiten Maulteils einen, zwei oder mehr Richtungsänderungsbereiche aufweist. Die Richtungsänderungsbereiche ändern dann die Ausrichtung des Maulteils in einer bestimmten Ebene und ermöglichen somit ein wirkungsvolles Umgreifen und/oder Hintergreifen des zu bearbeitenden Knochens, wie eines menschlichen Fingerknochens.

Damit bei Parallelausrichtung der Repositionszange zum Knochen die einzelnen Maulteile möglichst eng am Knochen entlanggeführt werden können, ist es von Vorteil, wenn an dem Richtungsänderungsbereich des zweiten Maulteils eine Richtungsänderung, eine Biegung oder einen Knick von ca. 75° bis ca. 115°, vorzugsweise ca. 85°, ca. 90° oder ca. 95° aufweist.

Dier Herstellung kann vereinfacht werden, wenn der Richtungsänderungsbereich spanabhebend, etwa gefräst, oder biegetechnisch geschaffen ist. Besonders bevorzugt ist hier eine Fräsvariante.

Die Freipräparation des Knochens kann besonders klein bleiben, wenn sich das erste Maulteil und/oder sich das zweite Maulteil vom Drehgelenk bis zum jeweiligen freien Ende kontinuierlich oder diskontinuierlich / abschnittsweise / stufenweise verjüngt bzw. verjüngen.

Wenn das erste Maulteil in der zur Schwenkebene parallelen Ebene (Parallelebene) zwischen dem Drehgelenk und dem freien Ende des ersten Maulteils einen, zwei oder mehr Ausrichtungsänderungsbereiche aufweist, so wird nur ein minimalinvasiver Eingriff an einem Finger, dessen Knochen behandelt werden soll, nötig. Dewr zu präparierende Bereich kann klein bleiben.

Es ist von Vorteil, wenn an dem Ausrichtungsänderungsbereich das erste Maulteil eine Richtungsänderung, eine Biegung oder einen Knick von ca. 75° bis 115°, vorzugsweise ca. 85°, ca. 90° oder ca. 95° aufweist.

Dabei ist es von Vorteil, wenn der Richtungsänderungsbereich und der Ausrichtungsänderungsbereich dieselbe Ausrichtungsveränderung/ Richtungsveränderung/Umlenkung an dem jeweiligen Maulteil hervorruft. Letztlich können die beiden Maulteile zumindest abschnittsweise zueinander symmetrisch oder sogar identisch ausgestaltet sein.

Ein vorteilhaftes Ausführungsbeispiel ist auch dadurch gekennzeichnet, dass das erste Maulteil einen endnahen Verbindungsteg und/oder einen drehgelenknahen Verbindungssteg aufweist. Die beiden Verbindungsstege verbinden dann die Schwenkebene mit der Parallelebene.

Dabei ist es von Vorteil, wenn der Verbindungssteg quer zur Schwenkebene ausgerichtet ist, vorzugsweise im Wesentlichen orthogonal oder in einem ca. 45°-Winkel. Vorteilhaft ist es hierbei, wenn der endnahe Verbindungssteg einen 90°-Winkel zum nächstgelegenen Maulteilabschnitt aufweist und der drehgelenknahe Verbindungssteg einen ca. 45°-Winkel zu dem jeweils nächstgelegenen Maulteilabschnitt aufweist.

Die Fertigung kann vereinfacht werden, wenn zwei der Ausrichtungsänderungsbereiche und/oder zumindest das dazwischen angeordnete Teilstück vollständig in der zur Schwenkebene parallelen Ebene, d.h. der Parallelebene, angeordnet ist.

Ferner ist es von Vorteil, wenn sich endseitig an dem endnahen Verbindungssteg ein Endstück anschließt, das vorzugsweise vollständig in der gemeinsamen Schwenkebene der beiden Maulteile liegt.

Es ist von Vorteil, wenn am ersten Maulteil und/oder dem zweiten Maulteil wenigstens ein orthogonal zum jeweiligen Maulteil abstehendes und auf das jeweils andere Maulteil weisendes / ausgerichtetes Halteelement vorhanden ist.

In Weiterführung dieses Gedankens ist es von Vorteil, wenn an einem Maulteil, etwa dem ersten Maulteil, zwei Halteelemente hintereinander in der Schwenkebene, vom Drehgelenk aus gesehen, angeordnet sind.

Das Halteelement kann als Zapfen, Dorn, Pin oder Stift ausgebildet sein und eine vorzugsweise kegelartige Ausprägung zumindest am freien Ende aufweisen.

Wenn in der Repositionszange vier Pins, zwei oben und zwei unten, eingesetzt sind, wird eine bessere Verankerung der Repositionszange am Knochen gewährleistet. Wenn die Pins ausgebildet sind, um in den Knochen hineinzudringen, ohne auf viel Widerstand zu stoßen, so wird die Verankerung noch weiter verbessert. Ein Abrutschen der Repositionszange am Knochen wird dadurch verhindert und eine stabilere Knochenposition gewährleistet.

Es ist von Vorteil, wenn der erste Stab neben dem zweiten Stab im Bereich des Drehgelenks angeordnet ist, also beide Stäbe in unterschiedlichen, zueinander parallelen Ebenen befindlich sind, oder die beiden Stäbe über einen Durchsteckschluss miteinander in Kontakt stehen. Ein Durchsteckschluss ist eine solche Verbindungsart, bei der der eine Stab durch Teilbereiche des anderen Stabes auf seinen beiden Seiten umgeben ist, also durch den anderen Stab hindurchgesteckt ist.

Es ist von Vorteil, wenn das erste Maulteil zumindest um zwei zueinander orthogonal stehende Ausrichtungsänderungsachsen/Biegeachsen eine bzw. zwei Ausrichtungsänderung(en)/Biegung(en) erfährt bzw. richtungsverändert bzw. gebogen ist.

Dabei ist es von Vorteil, wenn jene Ebenen, in denen die beiden Ausrichtungsänderungsachsen/Biegeachsen aufgenommen sind, im Wesentlichen soweit voneinander beabstandet sind, wie ein Drittel bis zwei Drittel, vorzugsweise die Hälfte der Breite eines zu behandelnden Knochens, quer zu dessen Längsrichtung gemessen.

Es ist zweckmäßig, wenn das Drehgelenk ein die Drehachse definierendes zylindrisches Lagerelement, wie einen Bolzen, einen Zapfen oder eine Schraube umfasst.

Das Verwenden von bekannten Griffabschnitten, etwa mit Verrasterungen oder Verriegelungen, lässt sich vorteilhafterweise einsetzen, wenn der erste Stab und/oder der zweite Stab eine Schweißnaht zwischen dem Drehgelenk und dem freien Ende des Maulteils aufweist. Die Schweißnaht kann auch auf der dem Maulteil abgewandten Seite des Drehgelenks vorhanden sein. Die Schweißnaht kann im MIG-, MAG-, oder WIG-Verfahren geschaffen worden sein.

Für die Belastbarkeit ist es von Vorteil, wenn das erste Maulteil als ein einstückiges Integralbauteil ausgebildet ist.

Hier ist es auch von Vorteil, wenn das zweite Maulteil als ein einstückiges Integralbauteil ausgebildet ist. Bei diesen zwei Unterformen wird auch die Fertigung vereinfacht.

Wenn die Länge der Maulteile von der Drehachse zum jeweiligen freien Ende der Maulteile und die Länge des Halteelements / der Halteelemente zum Greifen eines 4 mm bis 14 mm dicken Zylinders abgestimmt ist, so ist der Einsatz der Knochenreposition effizient möglich.

Für ein sicheres Halten des Knochens zuträglich ist es, wenn die Enden der beiden Maulteile als Vierpunktlager zum Halten eines Knochens ausgebildet sind.

Es soll nicht unerwähnt bleiben, dass es von Vorteil ist, dass das erste oder zweite Maulteil aus einer Aneinanderreihung unterschiedlicher, gerader Abschnitte aufgebaut ist und keine kontinuierliche Biegung in drei Dimensionen aufweist. Die Erfindung betrifft auch ein Verfahren zum Kontaktieren eines Knochens, etwa eines Fingerknochens, und/oder einer Knochenverbindungsplatte mit einer erfindungsgemäßen Repositionspfanne. Das Verfahren kann eine präoperative Planung einsetzen. Neben der Durchführung von Standardaufnahmen in der Neutralstellung etwa einer zu behandelnden Hand im A/P- und seitlichen Strahlengang, könnte bei intraartikulären Frakturen zur weiteren Abklärung eine hochauflösende Computertomografie durchgeführt werden.

Die Lagerung des Patienten wird in Rückenlage auf einem Operationstisch bewirkt. Jene zu operierende Hand wird in Pronationsstellung des Unterarms auf dem seitlichen Handtisch positioniert. Es wird ein lateraler Zugang geschaffen. Die Eröffnung erfolgt über eine seitliche, gerade Inzision, beginnend auf Höhe des MCP-Gelenks bis zum PIP-Gelenk.

Als nächster Schritt, der genauso wie die vorhergehend beschriebenen Schritte und die nachfolgend beschriebenen Schritte in genau dieser zeitlichen Reihenfolge erfolgt, wird die Darstellung der Fraktur ermöglicht. Nach der Hautinzision werden zunächst die radial, ulnar und median verlaufenden Nerven lokalisiert. Die schräg verlaufenden Fasern eines Seitenbandes werden mit zwei Wundhaken abgehalten. Das Periost wird ausschließlich unmittelbar neben der Fraktur angehoben, um einer Narbenbildung, der Verklebung der Bänder sowie der Devaskularisierung der Knochenfragmente vorzubeugen.

Als nächster Schritt steht die Reposition der Fraktur an. Neben der manuellen Reposition der Fraktur durch den Operateur, kann entweder eine ins Linos-System integrierte kleine Backhausklemme oder eine speziell für Fingerfrakturen entwickelte Repositionszange verwendet werden. Die Repositionszange spielt dann alle ihre zahlreichen Vorteile aus.

Nachfolgend findet eine Auswahl einer Osteosyntheseplatte mit anschließender Einbringung statt. Die Versorgung im vorliegenden Indikationsfall wird beispielhaft mit einer 0,8 mm T-Platte vorgenommen. Die Wahl der Osteosyntheseplatte erfolgt immer gemäß dem Frakturverlauf und der Patientenanatomie. Die Osteosyntheseplatte wird ggf. mit Hilfe zwei Plattenbiegezangen an die anatomischen Gegebenheiten angepasst. Die jeweilige Plattenbiegezange ist eine besondere Unterform der Repositionszange, nämlich eine solche, die am oberen Maulteil / der oberen Branche einen einzelnen als Halteelement agierenden Zapfen einsetzt, der auf die Innenkontur eines Lochs der Platte angepasst ist.

Es kann eine temporäre Fixierung der Platte mit 0,9 mm dicken K-Drähten erfolgen. Hierzu stehen spezielle K-Draht-Löcher zur Verfügung. Alternativ kann auch eine Linos-Plattenhaltezange verwendet werden.

Im nun nachfolgenden nächsten Schritt findet das Bohren eines ersten Kernlochs statt. Zum Sichern des Verschlusses des Frakturspaltes bietet es sich an, die Reihenfolge der Schraubenimplantationen derart zu wählen, dass eine Kompressionsbohrung genutzt werden kann. Aus diesem Grund werden zunächst die dem Langloch entfernten Bohrungen besetzt. Hierfür wird zunächst das Kernloch mit Hilfe der Bohrlehre und dem entsprechenden Kernlochbohrer gebohrt. Das Linos-System erlaubt in sämtlichen Plattenlöchern die Verwendung von Standard- als auch multidirektional winkelstabilen "Smart Drive-Schrauben" der Durchmesser 1,5 mm, 2 mm, 2,3 mm.

Danach erfolgt die Bestimmung der Schraubenlänge. Die korrekte Schraubenlänge wird mit einem Tiefenmesser bestimmt, der durchgängig für die Schraubendurchmesser 1,5, 2,0 und 2,3 mm verwendet werden kann. Hier wird eine Farbcodierung auf die Schraubendurchmesser abgestimmt. Danach erfolgt das Einbringen der ersten Schraube. Die Platte wird zunächst mit einer 2,0 mm Standard-"Smart Drive-Schraube" fixiert. Hierfür wird die Schraube mit dem entsprechenden farbcodierten Schraubendreher, der für die Durchmesser 1,5 oder 2,0 oder 2,3 mm verwendet wird, aufgenommen und eingedreht. Nun wird die zweite Schraube nach der oben beschriebenen Technik eingebracht, wahlweise kann zur Erhöhung der Stabilität ggf. eine winkelstellige Schraube verwendet werden. Die korrekte Position der Platte wird unter einer Röntgenkontrolle sichergestellt. Danach erfolgt das Einbringen der Kompressionsschraube. Nach erfolgreicher Implantation der ersten Schrauben wird nun zum sicheren Verschluss des Frakturspalts die Kompressionsschraube in das Langloch eingebracht. Es werden Standardschrauben der Durchmesser 1,5, 2,0 oder 2,3 mm verwendet. Hierzu wird die Kompressionsbohrbuchse in das offene Arbeitsende der Bohrlehre von unten eingeklickt. Die Pfeile auf der Kompressionsbohrbuchse zeigen dann beim Bohren in Richtung der Fraktur. Analog zur ersten Schraube wird das Kernloch gebohrt und die Länge der Schraube bestimmt.

Danach erfolgt der Verschluss des Frakturspalts. Beim Eindrehen gleitet die "Smart Drive-Standardschraube" über die in das Langloch integrierte schiefe Ebene in Richtung des Frakturspalts und schließt diesen.

Danach werden weitere Schrauben angebracht. Zur Erreichung einer ausreichenden, frühfunktionalen Stabilität werden weitere Plattenlöcher mit Schrauben besetzt. Die Vorgehensweise hierfür entspricht den vorgenannten Schritten. Die Anzahl sowie die Auswahl des Schraubendurchmessers und -typs erfolgt in Abhängigkeit der spezifischen Anatomie des Patienten und der notwendigen Stabilität.

Nun erfolgt der Wundverschluss. Nach dem sicheren Verschließen des Periosts erfolgt eine Hautnaht, mit nicht resorbierbarem Nahtmaterial.

Danach ist noch eine Nachbehandlung durchzuführen. Postoperativ wird der versorgte Finger mit einer Bandage am Finger fixiert, um seitlichen, auf den Finger wirkenden Kräfte zu neutralisieren.

In der Regel stellt sich der Patient fünf und zehn Tage nach der Operation zu einer Nachuntersuchung vor.

Unmittelbar nach dem operativen Eingriff kann der Patient mit Funktionsübungen beginnen.

Eine Metallentfernung ist insbesondere dann indiziert, wenn es zu weichgewebigen Irritationen kommt oder sich eine verminderte Beweglichkeit der Gelenke und des Fingers einstellt.

Es ist möglich, dass die Halteelemente, wie Pins oder Dornen als separate Bauteile in das ansonsten integral gefertigte Maulteil eingesetzt sind, bspw. in Löcher über eine Presspassung.

Es wird somit eine Repositionszange vorgestellt, die zwei durch ein Gelenk schwenkbar miteinander verbundene Schenkel aufweist, wobei an einem Maulabschnitt eines jeden Schenkels jeweils ein Kraftangriffspunkt definierbar ist, an dem bei rein starrkörpermechanischer Betrachtung beim Greifen eines Gegenstandes durch die Repositionszange eine einzelne resultierende Kraft angreift, deren Wirkung der Wirkung aller von dem Schenkel auf den Gegenstand wirkender Kräfte entspricht und wobei die beiden resultierenden Kräfte beider Schenkel betragsmäßig gleich groß sind , wobei die Kraftangriffspunkte unterschiedlich weit vom Gelenk entfernt sind, und zumindest ein Schenkel zumindest abschnittsweise quer zu einer, vom Gelenk und den Kraftangriffspunkten definierten, Ebene verläuft.

Die Erfindung wird nachfolgend mit Hilfe einer Zeichnung näher erläutert, in der ein erstes Ausführungsbeispiel dargestellt ist. Es zeigen:
- Fig. 1: eine Seitenansicht auf eine Repositionszange gemäß einer ersten Ausführungsform,
- Fig. 2: eine Ansicht von unten auf die Repositionszange aus Fig. 1,
- Fig. 3: eine Ansicht auf die Repositionszange aus Fig. 1 von oben,
- Fig. 4: eine Ansicht auf die Repositionszange aus Fig. 1 von vorne,
- Fig. 5: eine Ansicht von vorne im Bereich der Vergrößerung V aus Fig. 4 auf die geschlossene Repositionszange,
- Fig. 6: eine Vergrößerung des Bereiches VI aus Fig. 1,
- Fig. 7: eine Vergrößerung des Bereiches VII, der die geschlossene Repositionszange darstellenden Fig. 1,
- Fig. 8: die Repositionszange der Fig. 1 bis 7 in einer geschlossenen und überlagerten geöffneten Position,
- Fig. 9: eine Seitenansicht des ersten Maulteils (untere Branche / unteres Maulteil),
- Fig. 10: eine Ansicht von oben auf das erste Maulteil aus Fig. 9,
- Fig. 11: eine Ansicht von vorne auf das erste Maulteil der Fig. 9 und 10,
- Fig. 12: eine Ansicht von hinten auf das erste Maulteil der Fig. 9 bis 11,
- Fig. 13: eine Ansicht von der Seite auf das zweite Maulteil (oberes Maulteil / obere Branche), und
- Fig. 14: eine Ansicht von oben auf das zweite Maulteil der Fig. 13

Die Figuren sind lediglich schematischer Natur und dienen nur dem Verständnis der Erfindung. Die gleichen Elemente sind mit denselben Bezugszeichen versehen. Solche Merkmale, die nicht als wesentlich dargestellt sind, sind rein optional und daher austauschbar.

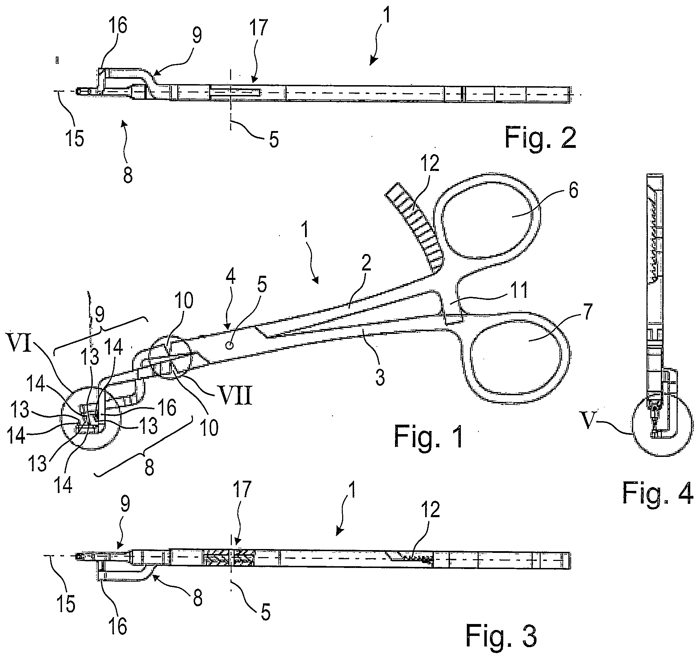

In Fig. 1 ist eine Repositionszange 1 nach einer ersten Ausführungsform dargestellt. Die Repositionszange 1 weist eine erste Stange 2 und eine zweite Stange 3 auf. Die erste Stange 2 ist mit der zweiten Stange 3 an einem Drehgelenk 4 verbunden. Das Drehgelenk 4 kann auch als Drehlager, Schwenklager oder Schwenkgelenk bezeichnet werden. Es beinhaltet eine Drehachse 5, die senkrecht durch die beiden Stangen 2 und 3 hindurch ragt und durch einen Bolzen gebildet wird. Die erste Stange 2 weist auf dem einen Ende einen Haltegriff 6 zum Aufnehmen eines Fingers einer Bedienperson auf, genauso wie die zweite Sange 3 auf derselben Seite einen Haltegriff 7 für einen anderen Finger der Bedienperson aufweist.

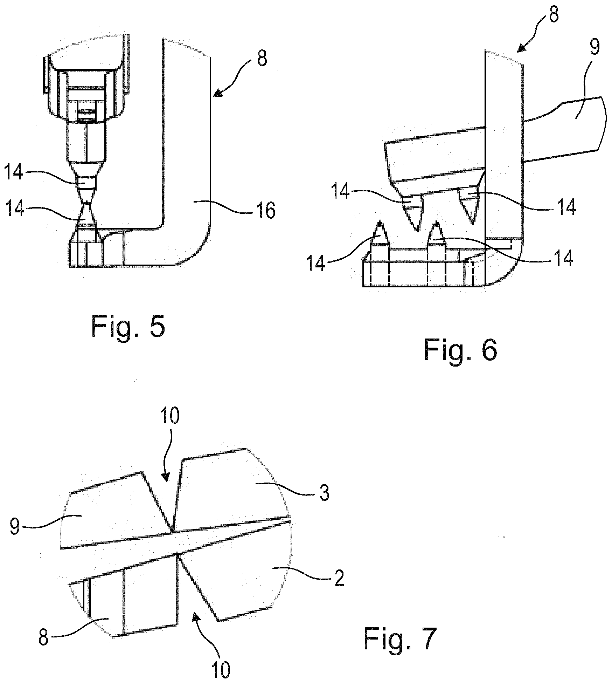

Die erste Stange 2 weist am anderen Ende, also dem Haltegriff 6 gegenüberliegend, ein erstes Maulteil 8 auf. Die zweite Stange 3 weist auf der gleichen Seite der Drehachse 5 ein zweites Maulteil 9 auf. Am drehgelenkseitigen Ende der beiden Maulteile 8 und 9 ist eine Kerbe 10 vorgesehen, um eine Schweißnaht aufzunehmen, die hier nicht dargestellt ist. Die Kerbe 10 ist besonders gut in der Fig. 7 zu erkennen.
Zurückkommend zu Fig. 1 sei erläutert, dass das erste Maulteil 8 das untere Maulteil, also die untere Branche ausbildet. Das zweite Maulteil 9 bildet die obere Branche, also das obere Maulteil aus. Es ist gut zu erkennen, dass am Haltegriff 6 ein Vorsprung 11 vorhanden ist, der mit einer Rastierung / Verrastung / Verrasterung 12 in Wirkkontakt gelangbar ist.

In Fig. 1 ist gut zu erkennen, dass Kontaktpunkte 13 in der Nähe der freien Enden der beiden Maulteile 8 und 9, nämlich solche Kontaktpunkte 13, geschaffen durch die Spitzen von Halteelementen 14, in einer gemeinsamen Schwenkebene bewegbar sind, in der ein durch die Drehachse 5 bestimmter Drehpunkt liegt und festzustellen ist, dass in dieser definierten Schwenkebene die Drehachse 5 senkrecht steht. In der Schwenkebene ist auch die Längsachse 15 der Repositionszange 1, die gleichzeitig auch die Längsachse der zweiten Stange 3 ist, eingebettet.
Ein gerader Abschnitt 16 ist in einer zur Schwenkebene parallelen Ebene, also einer Parallelebene, komplett eingebettet. Die beiden Ebenen sind zwischen 6 und 10 mm, vorzugsweise 7,5 mm voneinander beabstandet.
In Fig. 2 und 3 ist ein Durchsteckschluss 17, zur Verbindung der beiden Stangen 2 und 3 angedeutet. Die gesamte Repositionszange 1 ist zwischen 140 und 145 mm lang.
Das erste Maulteil 8 wechselt aus der Schwenkebene heraus, in die Parallelebene hinein und wieder zurück in die Schwenkebene. Die Kontaktpunkte 13 sind nahezu übereinander angeordnet, oder zumindest in derselben Schwenkebene befindlich.
Die Halteelemente 14 sind als Dorne ausgestaltet. Dies ist auch gut in den Fig. 5 und 6 zu erkennen.

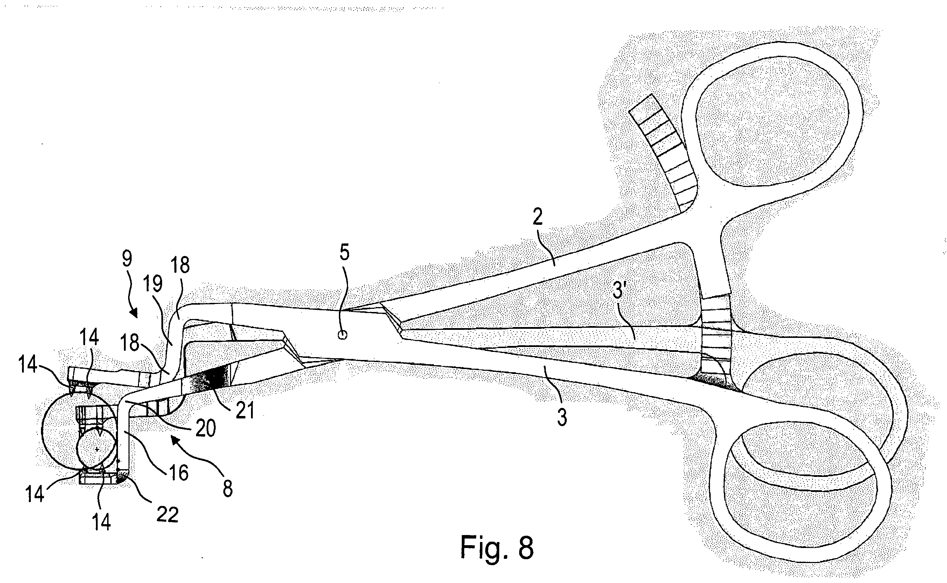

Die Halteelemente 14 können in das jeweilige Maulteil 8 oder 9 eingepresst und/oder eingeschweißt sein. Die beiden Maulteile 8 und 9 sind gehärtet, vorzugsweise mit 46 +/-2 HRC gehärtet. In Fig. 8 ist gut zu erkennen, dass sich der Mittelpunkt eines nahezu 2,5-mal so großen Zylinders, welcher von der Repositionszange 1 greifbar ist, um 10 % weiter von der Drehachse 5 entfernt. Die Stange 3 verlagert sich beim Schließen zur Position 3'. Das zweite Maulteil 9 weist zwei ca. 89°-Winkel auf der Unterseite bemessene bzw. 98°-Winkel auf der Oberseite bemessene Richtungsänderung an der Stelle eines Richtungsänderungsbereiches 18 auf. Ein zweiter, weiter unten und weiter distal angeordneter Richtungsänderungsbereich 18 weist einen ca. 90°-Winkel auf mit einem inneren Radius von ca. 2 mm und einem äußeren Radius von ca. 5 mm. Dazwischen ist ein ungebogenes Geradteil 19 vorhanden.

Das erste Maulteil 8 weist zwei Ausrichtungsänderungsbereiche 20 auf. Dazwischen ist der gerade Abschnitt 16 in der Parallelebene befindlich. Auf der Drehgelenkseite des drehgelenknäheren Ausrichtungsänderungsbereiches 20 schließt sich ein drehgelenknaher Verbindungssteg 21 an.

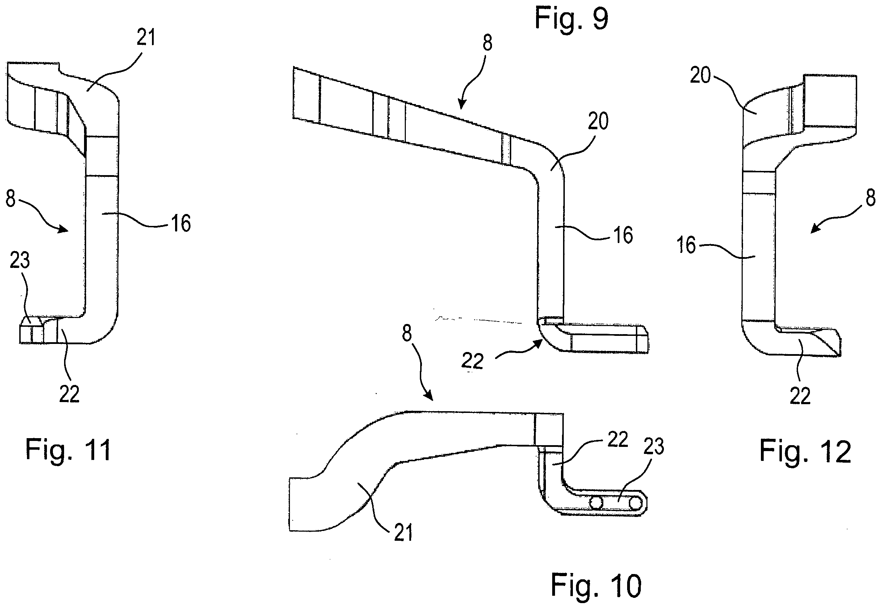

Ein endnaher Verbindungssteg 22 schließt sich an die endnahere der beiden Ausrichtungsänderungsbereiche 20 an. In Zusammenschau der Fig. 9 und 10 setzt sich für den Fachmann ein räumliches Bild zusammen, was durch die Fig. 11 und 12 noch unterstützt wird.

An dem endnahen Verbindungssteg 22 schließt ein Endteil 23 an. Wie in Fig. 10 gut zu erahnen, liegt im Anschlussbereich an dem drehgelenknahen Verbindungssteg 21 zu den dort vorhandenen Schenkelabschnitten ein Winkel von ungefähr 135° bzw. ungefähr 125° vor.

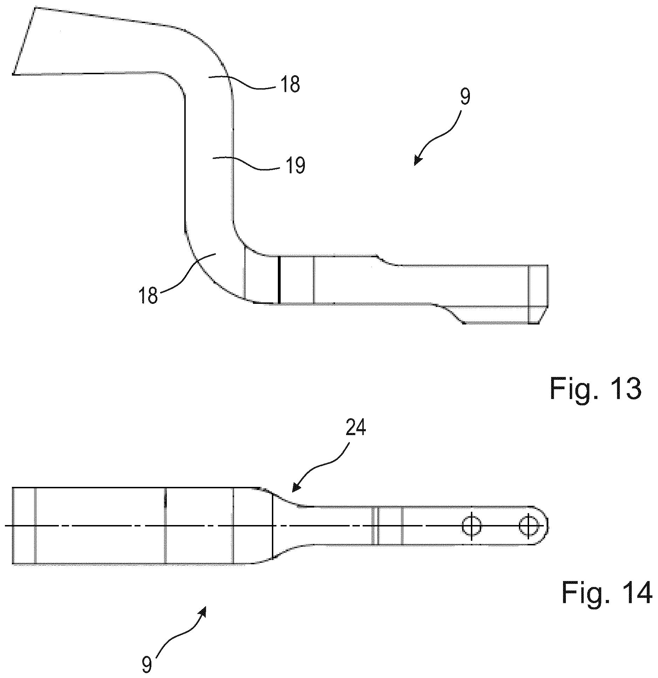

In den Fig. 13 und 14 ist das zweite Maulteil 9 näher dargestellt, wobei es einen Verjüngungsabschnitt 24 gibt. Die Halteelemente 14 sind in den Darstellungen der Fig. 9 bis 14 nicht enthalten.

### Bezugszeichenliste

- 1: Repositionszange
- 2: erste Stange
- 3: zweite Stange
- 4: Drehgelenk
- 5: Drehachse
- 6: Haltegriff
- 7: Haltegriff
- 8: erstes Maulteil
- 9: zweites Maulteil
- 10: Kerbe
- 11: Vorsprung
- 12: Verrasterung
- 13: Kontaktpunkt
- 14: Halteelement
- 15: Längsachse
- 16: gerader Abschnitt
- 17: Dursteckschluss
- 18: Richtungsänderungsbereich
- 19: Geradteil
- 20: Ausrichtungsänderungsbereich
- 21: drehgelenknaher Verbindungssteg
- 22: endnaher Verbindungssteg
- 23: Endteil
- 24: Verjüngungsabschnitt

## Patentansprüche

1. Repositionszange (1) mit einer ersten Stange (2) und einer zweien Stange (3), die an einem eine Drehachse (5) definierenden Drehgelenk (4) zueinander verschwenkbar gelagert sind, wobei die erste Stange (2) auf einer Seite des Drehgelenks (4) ein erstes Maulteil (8) aufweist, und die zweite Stange (3) auf der gleichen Seite des Drehgelenks (4) ein zum Zusammenwirken mit dem ersten Maulteil (8) vorgesehenes zweites Maulteil (9) aufweist, wobei an den beiden Maulteilen (8 und 9) für ein Kontaktieren eines Knochens oder einer Knochenplatte vorbereitete Kontaktpunkte (13) definiert sind, **dadurch gekennzeichnet, dass** die Kontaktpunkte (13) in einer gemeinsamen Schwenkebene, die das Drehgelenk beinhaltet, bewegbar sind, in der die Drehachse (5) senkrecht steht und wenigstens eines der Maulteile (8, 9) in Richtung einer zur Schwenkebene parallelen Ebene abschnittsweise versetzt ist.

2. Repositionszange (1) nach Anspruch 1, **dadurch gekennzeichnet, dass** nur eine der beiden Maulteile (8 oder 9) in Richtung der zur Schwenkebene parallelen Ebene verlaufend ausgerichtet ist.

3. Repositionszange (1) nach Anspruch 1 oder 2, **dadurch gekennzeichnet, dass** ein Abschnitt (16) des ersten Maulteils (8) in der zur Schwenkebene parallelen Ebene verlaufend angeordnet ist.

4. Repositionszange (1) nach einem der Ansprüche 1 bis 3, **dadurch gekennzeichnet, dass** das erste Maulteil (8) eine untere Branche und das zweite Maulteil (9) als eine obere Branche ausgestaltet ist, wobei ein freies Ende der unteren Branche in der Schwenkebene betrachtet unterhalb der oberen Branche angeordnet ist.

5. Repositionszange (1) nach einem der Ansprüche 1 bis 4, **dadurch gekennzeichnet, dass** das zweite Maulteil (9) vollständig oder überwiegend in der Schwenkebene angeordnet ist.

6. Repositionszange (1) nach einem der Ansprüche 1 bis 5, **dadurch gekennzeichnet, dass** das zweite Maulteil (9) in der Schwenkebene zwischen dem Drehgelenk (4) und dem freien Ende des zweiten Maulteils (9) einen, zwei oder mehr Richtungsänderungsbereiche (18) aufweist.

7. Repositionszange (1) nach Anspruch 6, **dadurch gekennzeichnet, dass** an dem Richtungsänderungsbereich (18) das zweite Maulteil (9) eine Richtungsänderung, eine Biegung oder einen Knick von ca. 75° bis ca. 115°, vorzugsweise ca. 85°, ca. 90° oder ca. 95° aufweist.

8. Repositionszange (1) nach einem der Ansprüche 6 oder 7, **dadurch gekennzeichnet, dass** der Richtungsänderungsbereich (18) spanabhebend, etwa gefräst, oder biegetechnisch geschaffen ist.

9. Repositionszange (1) nach einem der Ansprüche 1 bis 8, **dadurch gekennzeichnet, dass** sich das erste Maulteil (8) und/oder das zweite Maulteil (9) vom Drehgelenk (4) bis zum jeweiligen freien Ende kontinuierlich oder stufenweise verjüngt/verjüngen.

10. Repositionszange (1) nach einem der Ansprüche 1 bis 9, **dadurch gekennzeichnet, dass** das erste Maulteil (8) in der zur Schwenkebene parallelen Ebene zwischen dem Drehgelenk (4) und dem freien Ende des ersten Maulteils (8) einen, zwei oder mehr Ausrichtungsänderungsbereiche (20) aufweist.

11. Repositionszange (1) nach Anspruch 10, **dadurch gekennzeichnet, dass** an dem Ausrichtungsänderungsbereich (20) das erste Maulteil (8) eine Richtungsänderung, eine Biegung oder einen Knick von ca. 75° bis ca. 115°, vorzugsweise ca. 85°, ca. 90° oder ca. 95° aufweist.

12. Repositionszange (1) nach einem der Ansprüche 7 bis 11, **dadurch gekennzeichnet, dass** der Richtungsänderungsbereich (18) und der Ausrichtungsänderungsbereich (20) dieselbe Ausrichtungsänderung in dem jeweiligen Maulteil (8 oder 9) hervorruft.

13. Repositionszange (1) nach einem der Ansprüche 1 bis 12, **dadurch gekennzeichnet, dass** die zweite Stange (3) mit dem zweiten Maulteil (9) in einer die Längsachse (15) der zweiten Stange (3) aufnehmenden Bearbeitungsebene, die deckungsgleich mit der Schwenkebene ist, angeordnet ist.

14. Repositionszange (1) nach Anspruch 13, **dadurch gekennzeichnet, dass** der Verbindungssteg (21, 22) quer zur Schwenkebene ausgerichtet ist, vorzugsweise im Wesentlichen orthogonal oder in einem ca. 45°-Winkel.

15. Repositionszange (1) nach einem der Ansprüche 10 bis 14, **dadurch gekennzeichnet, dass** zwei der Ausrichtungsänderungsbereiche (20) und/oder zumindest das dazwischen angeordnete Teilstück vollständig in der zur Schwenkebene parallelen Ebene angeordnet ist.

## Claims

1. Reduction forceps (1) comprising a first rod (2) and a second rod (3), which are pivotally mounted relative to each other on a rotation joint (4) defining an axis of rotation (5), the first rod (2) comprising a first jaw part (8) on one side of the rotation joint (4) jaw part and the second rod (3) comprising, on the same side of the rotation joint (4), a second jaw part (9) which is provided to cooperate with the first jaw part (8), contact points (13) which are provided for contacting a bone or a bone plate being defined on both jaw parts (8, 9), **characterised in that** the contact points (13) are movable in a common pivoting plane, which contains the rotation joint and in which the axis of rotation (5) extends perpendicularly, and at least one of the jaw parts (8, 9) is offset in portions in the direction of a plane extending in parallel with the pivoting plane.

2. Reduction forceps (1) according to claim 1, **characterised in that** only one of the two jaw parts (8 or 9) is oriented so as to extend in the direction of the plane that is parallel to the pivoting plane.

3. Reduction forceps (1) according to either claim 1 or claim 2, **characterised in that** a portion (16) of the first jaw part (8) is arranged so as to extend in the plane that is parallel to the pivoting plane.

4. Reduction forceps (1) according to any of claims 1 to 3, **characterised in that** the first jaw part (8) is configured as a lower branch and the second jaw part (9) is configured as an upper branch, a free end of the lower branch being arranged below the upper branch, when viewed in the pivoting plane.

5. Reduction forceps (1) according to any of claims 1 to 4, **characterised in that** the second jaw part (9) is arranged fully or mostly in the pivoting plane.

6. Reduction forceps (1) according to any of claims 1 to 5, **characterised in that** the second jaw part (9) comprises one, two or more direction-change regions in the pivoting plane between the rotation joint (4) and the free end of the second jaw part (9).

7. Reduction forceps (1) according to claim 6, **characterised in that** in the direction-change region (18), the second jaw part (9) has a change of direction, a bend or a kink of from approximately 75° to approximately 115°, preferably approximately 85°, approximately 90° or approximately 95°.

8. Reduction forceps (1) according to either claim 6 or claim 7, **characterised in that** the direction-change region (18) is produced by means of a material-removing process, for example milling, or by means of a bending process.

9. Reduction forceps (1) according to any of claims 1 to 8, **characterised in that** the first jaw part (8) and/or the second jaw part (9) taper, continuously or in stages, from the rotation joint (4) to the respective free ends.

10. Reduction forceps (1) according to any of claims 1 to 9, **characterised in that** the first jaw part (8) comprises one, two or more orientation-change regions (20) between the rotation joint (4) and the free end of the first jaw part (8) in the plane extending in parallel with the pivoting plane.

11. Reduction forceps (1) according to claim 10, **characterised in that** in the orientation-change region (20), the first jaw part (8) has a change of direction, a bend or a kink of from approximately 75° to approximately 115°, preferably approximately 85°, approximately 90° or approximately 95°.

12. Reduction forceps (1) according to any of claims 7 to 11, **characterised in that** the direction-change region (18) and the orientation-change region (20) cause the same change of orientation in the respective jaw parts (8 or 9).

13. Reduction forceps (1) according to any of claims 1 to 12, **characterised in that** the second rod (3) comprising the second jaw (9) is arranged in a working plane which includes the longitudinal axis (15) of the second rod (3) and which is congruent with the pivoting plane.

14. Reduction forceps (1) according to claim 13, **characterised in that** the connecting piece (21, 22) is oriented transversely to the pivoting plane, preferably substantially orthogonally thereto or at an angle of approximately 45°.

15. Reduction forceps (1) according to any of claims 10 to 14, **characterised in that** two of the orientation-change regions (20) and/or at least the part arranged therebetween are arranged entirely in the plane extending in parallel with the pivoting plane.

## Revendications

1. Pince de repositionnement (1) avec une première tige (2) et une seconde tige (3) qui sont logées de manière pivotante l'une par rapport à l'autre sur une articulation tournante (4) définissant un axe de rotation (5), la première tige (2) présentant, sur un côté de l'articulation tournante (4), une première partie de mâchoire (8) et la seconde tige (3) présentant, sur le même côté de l'articulation tournante (4), une seconde partie de mâchoire (9) prévue pour coagir avec la première partie de mâchoire (8), des points de contact (13) préparés pour entrer en contact avec un os ou une plaque osseuse étant définis sur les deux parties de mâchoire (8 et 9), **caractérisée en ce que** les points de contact (13) peuvent être déplacés dans un plan de pivotement commun qui contient l'articulation tournante, dans lequel l'axe de rotation (5) est perpendiculaire et au moins l'une des parties de mâchoire (8, 9) est en déport par sections en direction d'un plan parallèle au plan de pivotement.

2. Pince de repositionnement (1) selon la revendication 1, **caractérisée en ce que** seule l'une des deux parties de mâchoire (8 ou 9) est orientée de manière à s'étendre en direction du plan parallèle au plan de pivotement.

3. Pince de repositionnement (1) selon la revendication 1 ou 2, **caractérisée en ce qu'**une section (16) de la première partie de mâchoire (8) est agencée de manière à s'étendre dans le plan parallèle au plan de pivotement.

4. Pince de repositionnement (1) selon l'une quelconque des revendications 1 à 3, **caractérisée en ce que** la première partie de mâchoire (8) est configurée comme une branche inférieure et la seconde partie de mâchoire (9) comme une branche supérieure, une extrémité libre de la branche inférieure étant agencée sous la branche supérieure, vu dans le plan de pivotement.

5. Pince de repositionnement (1) selon l'une quelconque des revendications 1 à 4, **caractérisée en ce que** la seconde partie de mâchoire (9) est agencée complètement ou principalement dans le plan de pivotement.

6. Pince de repositionnement (1) selon l'une quelconque des revendications 1 à 5, **caractérisée en ce que** la seconde partie de mâchoire (9) présente, dans le plan de pivotement entre l'articulation tournante (4) et l'extrémité libre de la seconde partie de mâchoire (9), une, deux ou plus zones de modification de direction (18).

7. Pince de repositionnement (1) selon la revendication 6, **caractérisée en ce que** la seconde partie de mâchoire (9) présente, sur la zone de modification de direction (18), une modification de direction, une courbure ou un coude d'environ 75° à environ 115°, de préférence d'environ 85°, d'environ 90° ou d'environ 95°.

8. Pince de repositionnement (1) selon l'une quelconque des revendications 6 ou 7, **caractérisée en ce que** la zone de modification de direction (18) est créée par enlèvement de copeaux, par exemple est fraisée, ou par pliage.

9. Pince de repositionnement (1) selon l'une quelconque des revendications 1 à 8, **caractérisée en ce que** la première partie de mâchoire (8) et/ou la seconde partie de mâchoire (9) se rétrécit/rétrécissent en continu ou graduellement de l'articulation tournante (4) à l'extrémité libre respective.

10. Pince de repositionnement (1) selon l'une quelconque des revendications 1 à 9, **caractérisée en ce que** la première partie de mâchoire (8) présente, dans le plan parallèle au plan de pivotement entre l'articulation tournante (4) et l'extrémité libre de la première partie de mâchoire (8), une, deux ou plus, zones de modification d'orientation (20).

11. Pince de repositionnement (1) selon la revendication 10, **caractérisée en ce que** la première partie de mâchoire (8) présente, sur la zone de modification d'orientation (20), une modification de direction, une courbure ou un coude d'environ 75° à environ 115°, de préférence d'environ 85°, d'environ 90° ou d'environ 95°.

12. Pince de repositionnement (1) selon l'une quelconque des revendications 7 à 11, **caractérisée en ce que** la zone de modification de direction (18) et la zone de modification d'orientation (20) suscitent la même modification d'orientation dans la partie de mâchoire (8 ou 9) respective.

13. Pince de repositionnement (1) selon l'une quelconque des revendications 1 à 12, **caractérisée en ce que** la seconde tige (3) est agencée avec la seconde partie de mâchoire (9) dans un plan d'usinage recevant l'axe longitudinal (15) de la seconde tige (3) qui est coïncident avec le plan de pivotement.

14. Pince de repositionnement (1) selon la revendication 13, **caractérisée en ce que** la nervure de liaison (21, 22) est orientée transversalement au plan de pivotement, de préférence sensiblement orthogonalement ou selon un angle d'environ 45°.

15. Pince de repositionnement (1) selon l'une quelconque des revendications 10 à 14, **caractérisée en ce que** deux des zones de modification d'orientation (20) et/ou au moins la pièce partielle agencée au milieu est complètement agencée dans le plan parallèle au plan de pivotement.
